# EUROPÄISCHE PATENTANMELDUNG

(11) **EP 1 431 265 A1**
(43) Veröffentlichungstag der Anmeldung: **23.06.2004**
(21) Anmeldenummer: 03028911.0
(22) Anmeldetag: 17.12.2003
(51) Int. Cl.: C07C 46/04, C07C 50/18

(54) **Verfahren zur Herstellung von Anthrachinon**

(30) Priorität: 21.12.2002 DE 10260549
(71) Anmelder: RÜTGERS Chemicals AG, 44579 Castrop-Rauxel (DE)
(72) Erfinder: Polaczek, Jerzy, Dr.Inz., 02-784 Warszawa (PL); Domanowski, Wojciech, Mgr.Inz., 03-226 Warszawa (PL); Pielichowski, Jan, Prof.Dr.Hab.Inz., 30-076 Krakow (PL); Machowska, Zofia, Mgr.Inz., 01-912 Warszawa (PL); Fuhrmann, Edgar, Dr., 44579 Castrop-Rauxel (DE); Talbiersky, Joerg, Dr., 46282 Dorsten (DE)
(74) Vertreter: COHAUSZ & FLORACK

(57) **Zusammenfassung**

In einem Verfahren zur Herstellung von Anthrachinon in der flüssigen Phase durch katalytische Oxidation von Anthracen behandelt man Anthracen in einem Carbonsäuremedium in Gegenwart eines organischen Metallsalzes und eines Aktivierungsmittels für das Metallsalz bei einer Temperatur von etwa 40 °C und mehr unter Einwirkung von Sauerstoff und Licht, anschließend mit Wasserstoffperoxid und isoliert das erhaltene Reaktionsprodukt aus dem Reaktionsgemisch, wobei Ausbeute und Reinheit des Anthrachinions deutlich gesteigert werden.

## Beschreibung

Die Erfindung betrifft ein Verfahren zur Herstellung von Anthrachinon durch katalytische Oxidation von Anthracen mit Sauerstoff in der Flüssigphase.

Anthrachinon ist ein wichtiger Ausgangsstoff in der Farbstoffherstellung. Unter der Bezeichnung Anthrachinon-Farbstoff wird ein große Gruppe äußerst lichtechter Farbstoffe verstanden.

Die Oxidation von Anthracen zu Anthrachinon gehört seit langem zum Stand der Technik und ist unter anderem in H.G. Franck, J.W. Stadelhofer, Industrielle Aromatenchemie, Springer Verlag 1987, beschrieben. Im allgemeinen wird dabei Anthrachinon durch katalytische Gasphasenoxidation aus Anthracen in Gegenwart eines molekularen Sauerstoff enthaltenden Gases hergestellt, wobei Schwermetalloxide als Katalysatoren eingesetzt werden. Die Herstellung von Anthrachinon mittels Gasphasenoxidation belastet durch freigesetzte Anthracen- und Anthrachinondämpfe die Umwelt erheblich. Nachteilig ist ferner, dass die Gasphasenoxidation eine niedrige Selektivität aufweist und während der Reaktion unerwünschte Nebenprodukte entstehen. Die Ausbeute an Anthrachinon nach der Gasphasenoxidation liegt je nach Katalysator bei 80 bis 95 %.

Neben der Gasphasenoxidation ist die Flüssigphasenoxidation, beispielsweise mit Dichromat oder Salpetersäure, ausgehend von festem Anthracen bekannt. Die Verwendung von Dichromat oder Salpetersäure in einer Flüssigphasenoxidation ist zur Vermeidung ökologischer Nachteile immer mit einer aufwendigen Wasseraufbereitung verbunden.

In der PL 0 295 761 wird ein Verfahren beschrieben, in dem Wasserstoffperoxid als Oxidationsmittel eingesetzt wird. Nachteilig an dem Verfahren ist jedoch, dass der Verbrauch an Wasserstoffperoxid so hoch ist, dass die Herstellung von Anthrachinon nicht mehr wirtschaftlich ist.

Die DE 32 07 572 A1 offenbart ein Verfahren zur Oxidation von reaktiven Aromaten (Anthracen) mit molekularem Sauerstoff in Carbonsäurelösung in Gegenwart eines Katalysators, der aus einer Kobaltverbindung und einer Mangan- und einer Bromverbindung besteht. Nach jedem Reaktionszyklus wird der Katalysator mit einem starken Oxidationsmittel wieder aktiviert. Starke Oxidationsmittel im Sinne diese Offenbarung sind Kaliumpermanganat, Ozon, Persäuren, Peroxidisulfate oder Chromsäure.

Der Erfindung liegt die Aufgabe zugrunde, ein Verfahren zur Erzeugung von Anthrachinon aus Anthracen bereitzustellen, das eine hohe Ausbeute an Anthrachinon umweltfreundlich und kostengünstig ermöglicht.

Gelöst wird diese Aufgabe durch ein Verfahren zur Herstellung von Anthrachinon durch katalytische Oxidation von Anthracen, in dem man Anthracen in der flüssigen Phase in Gegenwart eines organischen Metallsalzes und eines Aktivierungsmittels für das Metallsalz bei einer Temperatur von etwa 40 °C und mehr unter Einwirkung von Sauerstoff und Licht behandelt, eine Behandlung des Reaktionsgemischs mit Wasserstoffperoxid vornimmt und das erhaltene Reaktionsprodukt aus dem Reaktionsgemisch isoliert.

In dem erfindungsgemäßen Verfahren erfolgt die Oxidation des Anthracens mit den sich bei der Sauerstoffoxidation von Carbonsäuren in situ bildenden Peroxysäuren.

Die erfindungsgemäße Oxidation in der flüssigen Phase verläuft langsam aber selektiv in etwa nach einer Kinetik erster Ordnung, obwohl weder das Ausgangsmaterial Anthracen noch das Produkt im Reaktionsmedium löslich ist. Dieser Umstand hat jedoch überraschender Weise keinen Einfluß auf den Reaktionsverlauf. Überraschend ist auch die hohe Selektivität. Es verbleibt als Nebenprodukt nur wenig Anthron, das noch zu Anthrachinon umgesetzt werden könnte.

Als Ausgangsmaterial kann ein übliches technisches Anthracen eingesetzt werden, wie es beispielsweise von der Rütgers Chemicals AG bereitgestellt wird. Dieses weist eine Reinheit von etwa 96% auf und enthält als Hauptverunreinigungen Phenanthren (2 Gew.%), Carbazol (< 1 Gew.%) und Fluoren (< 1 Gew.%). Dieses Ausgangsmaterial Anthracen liegt im festen Zustand vor und kann so der Reaktion zugeführt werden.

Das Carbonsäuremedium kann gebildet werden durch Ameisensäure, Essigsäure, Eisessig, Propionsäure, Isopropionsäure, deren Gemische und weitere niedere organische Mono- oder Dicarbonsäuren und deren Säureanhydride. Geeignete Dicarbonsäuren sind bespielsweise Adipinsäure, Maleinsäure und Phthalsäure. Die Säuren können gegebenfalls in organischen Lösungsmitteln vorliegen. Gemäß einer besonders bevorzugten Ausführungsform ist das Carbonsäuremedium Eisessig. Das Carbonsäuremedium soll möglichst wasserfrei sein. Ein Wassergehalt von bis zu etwa 1% im Carbonsäuremedium ist unschädlich.

Das erfindungsgemäß eingesetzte katalytische System enthält ein aktiviertes organisches Metallsalz. Geeignete organische Metallsalze sind die Metallsalze der oben genannten, das Carbonsäuremedium bildenden Carbonsäuren. Zur Metallsalzbildung besonders geignete Metalle sind Übergangsmetalle in höherer Oxidationsstufe, +III, IV, V, VI und VII. Bevorzugte Salze sind solche von Mangan, Kobalt, Molybdän, Nickel, Zirkonium und Hafnium der zuvorgenannten Oxidationszahlen. Besonders bevorzugt sind Mangan und Kobalt.

Bevorzugte organische Metallsalze sind beispielsweise Mangan- und Kobaltacetat oder Mangancarbonat.

Die Metallsalze werden vorzugsweise in Konzentrationen von 0,0005 bis 0,2 mol/L, vorzugsweise 0,002 bis 0,05 mol/L eingesetzt.

Die Aktivierung des Metallsalzes erfolgt durch ein geeignetes Aktivierungsmittel. Aktivierungsmittel sind beispielsweise Bromide, Jodide oder Chloride. Besonders bevorzugt sind Bromide. Die Aktvierungsmittel in Form der zuvor genannten Halogenide können als Metallsalz oder organisches Halogenid eingesetzt werden. Geeignete Metallhalogenide sind beispielsweise die Alkalimetall- und Erdalkalimetallbromide und Ammoniumbromid. Geeignete organische Bromide sind beispielsweise Trimethyl-tert.butylammoniumbromid, Methintribromid (Bromoform), Acetylentetrabromid und Phenylbromid. Besonders bevorzugt als Aktivierungsmittel für das Metallsalz sind Natriumbromid und Ammoniumbromid

Das Aktivierungsmittel kann im Reaktionsgemisch in einer Konzentration von 0,001 bis 0,5 mol/L, vorzugsweise 0,002 bis 0,1 mol/L, enthalten sein.

Bevorzugt werden das aktivierte organische Metallsalz oder ein Gemisch aus Metallsalzen und das Aktivierungsmittel in einem Gewichtsverhältnis von 0,8:1 bis 1:0,8, besonders bevorzugt etwa 1:1 eingesetzt.

Der zur Reaktion eingesetzte Sauerstoff wird vorzugsweise gasförmig in das Reaktionsgemisch eingeleitet. Der Sauerstoffgehalt des Gases liegt über 60 Gew.%, vorzugsweise wird im wesentlichen reiner Sauerstoff eingesetzt.

Das erfindungsgemäße Verfahren kann in einem üblichen Rührreaktor batchweise oder kontinuierlich durchgeführt werden.

Nach dem erfindungsgemäßen Verfahren werden Anthracen und das katalytische System in dem Carbonsäuremedium suspendiert. Dies kann bei Raumtemperatur erfolgen. Unter Zufuhr von gasförmigem Sauerstoff und unter Lichtzutritt kann das erfindungsgemäße Verfahren in einem Temperaturbereich von 40 bis 100 °C, vorzugsweise 55 bis 85 °C, 3 bis 24 Stunden lang durchgeführt werden. Durch diese Verfahrensweise kann eine Ausbeute von 81 % Anthrachinon, bezogen auf das ursprünglich eingesetzte Anthracen, erzielt werden. Der Reinheitsgrad des so gewonnenen Anthrachinons liegt bei über 99,2 %.

Für die hohe Ausbeute an Anthrachinon im erfindungsgemäßen Verfahren erscheint die überraschende Wirkung des Wasserstoffperoxids bei vergleichsweise niedriger Temperatur von Bedeutung. Dazu wird das Reaktionsgemisch vor der Abtrennung des Reaktionsprodukts mit Wasserstoffperoxid behandelt. Die Dauer der Behandlung des Reaktionsgemischs mit Wasserstoffperoxid vor der Abtrennung des Reaktionsprodukts kann 0,5 bis 3 Stunden, vorzugsweise etwa 2 Stunden, betragen. Die Behandlung kann bei 18 bis 60 °C, vorzugsweise bei etwa 50 °C, durchgeführt werden. Wasserstoffperoxid kann dem Reaktionsgemisch in Form einer hochkonzentrierten Lösung zugesetzt werden. Die Konzentration der Lösung des Wasserstoffperoxids kann beispielsweise etwa 20% betragen. Im Reaktionsgemisch kann die Konzentration an H₂O₂ in einem Bereich von 0,7 bis 2 Gew.%, vorzugsweise über etwa 1%, bezogen auf die Masse des Reaktionsgemischs, gehalten werden.

Dadurch kann eine Ausbeute an Anthrachinon von über 94 Gew.% erlangt werden. Im Produkt sind dann nur noch Spuren von nicht umgesetztem Anthracen und kleine Mengen an 9-Anthron und Phenanthren (2 Gew.%) nachzuweisen.

Wasserstoffperoxid ist kein starkes Oxidationsmittel im Sinne der DE 32 07 572 A1. Es ist nicht zur Reaktivierung des Katalysators geeignet.

Da alle Nebenprodukte der Reaktion eine relativ gute Löslichkeit in Carbonsäure aufweisen, können diese durch Umkristallisieren vollständig entfernt werden. Die Reinheit des erfindungsgemäß erhaltenen Anthrachinons ist höher als 99,5%.

Das Produkt kann nach der Oxidationsreaktion durch Sedimentation, Zentrifugieren und Filtration gemäß bekannten Verfahren vom Reaktionsgemisch abgetrennt werden. Anschließend kann das Reaktionsprodukt einer gaschromatographischen Analyse zur Überprüfung der Reinheit unterzogen werden.

Beispielsweise wird mit dem erfindungsgemäßen Verfahren nach einer 24stündigen Reaktion bei einer Temperatur von 100 °C Anthrachinon mit einer Ausbeute von 86 Gew.% gewonnen. Das Reaktionsprodukt enthält weiterhin Phenanthren, 9-Hydroxy-10-acetoxyanthracen, 9-Anthron sowie kleinere Mengen von nicht umgesetztem Anthracen und 9-Acetoxyanthracen. Das aus dem Rohanthrachinon umkristallisierte Anthrachinon hat einen Reinheitsgrad von über 99,2 Gew.%. Die Ausbeute der Reaktion beträgt, bezogen auf das eingesetzte Anthracen, 81 %.

Das folgende Beispiel dient der weiteren Erläuterung der Erfindung.

### Beispiel 1

In einen mit magnetischem Rührer, Thermometer, Sauerstoffzufuhr und Rückflusskühler versehenen Dreihalsrundkolben eines Fassungsvermögens von 150 ml werden 50 ml Essigsäure (rein), 2,5 g technisches Anthracen, 0,08 g Mangancarbonat (rein) und 0,08 g Natriumbromid eingeführt. Das Gemisch wird unter Rühren und kontinuierlicher Begasung mit Sauerstoff (10 ml/min) auf 100 °C erwärmt und 24 Stunden lang oxidiert. Das Reaktionsgemisch wird auf seine chemische Zusammensetzung gaschromatographisch untersucht (Tab. 1). Nachdem der Anteil an nicht umgesetztem Anthracen unter 1 Gew.% gesunken ist, gilt die Reaktion als beendet. Danach werden 10 ml einer 30%igen Wasserstoffperoxidlösung zum Reaktionsgemisch gegeben und bei einer Temperatur von 50 °C weitere zwei Stunden lang gerührt. Nach dem Abkühlen des Reaktionsgemischs auf Raumtemperatur werden 3 g des gewonnenen Rohanthrachinons abfiltriert und aus 30 ml Essigsäure umkristallisiert. Das als Endprodukt gewonnene Anthrachinon (2,9 g) hat eine Reinheit von 99,6 %. Die Gesamtausbeute an Anthrachinon beträgt im Vergleich mit der theoretischen Ausbeute 95 %.

Die folgende Tabelle zeigt die Veränderung der Zusammensetzung des Reaktionsgemisches im Verlauf der Reaktion über einen Zeitraum von 26 Stunden.

| Zeit [h] | Zusammensetzung des Produkts, Gew.% | | | | | |
|---|---|---|---|---|---|---|
| | Anthracen | Anthrachinon | Anthron | 9-Hydroxy-10-acetoxyanthracen | 9-Acetoxyanthracen | Phenanthren |
| 0 | 96 | 0 | 0 | 0 | 0 | 2 |
| 5 | 46 | 21 | 7 | 19 | 5 | 2 |
| 10 | 19 | 44 | 8 | 21 | 5 | 2 |
| 15 | 5 | 66 | 8 | 15 | 3 | 2 |
| 21 | 2 | 74 | 7 | 13 | 2 | 2 |
| 24^{a)} | < 1 | 86 | 5 | 8 | < 1 | 2 |
| 26 | Spuren | 97 | < 1 | Spuren | Spuren | 2 |

| | | | | | | |
|---|---|---|---|---|---|---|
| (a) Zugabe von H₂O₂ | | | | | | |

## Patentansprüche

1. Verfahren zur Herstellung von Anthrachinon durch katalytische Oxidation von Anthracen, **dadurch gekennzeichnet, dass** man Anthracen in der flüssigen Phase in einem Carbonsäuremedium in Gegenwart eines organischen Metallsalzes und eines Aktivierungsmittels für das Metallsalz bei einer Temperatur von etwa 40 °C und mehr unter Einwirkung von Sauerstoff und Licht behandelt, vor der Isolierung des Reaktionsprodukts das Reaktionsgemisch mit Wasserstoffperoxid behandelt und das Reaktionsprodukt aus dem Reaktionsgemisch isoliert.

2. Verfahren nach Anspruch 1, **dadurch gekennzeichnet, daß** man die Behandlung mit Wasserstoffperoxid bei einer Temperatur des Reaktionsgemischs von bis zu etwa 60 °C durchführt.

3. Verfahren nach Anspruch 1 oder 2, **dadurch gekennzeichnet, dass** man die Konzentration des Wasserstoffperoxids im Reaktionsgemisch auf über etwa 1 Gew.% während der Reaktion hält.

4. Verfahren nach Anspruch 1, **dadurch gekennzeichnet, dass** man als organisches Metallsalz ein Mangan- oder Kobaltacetat, Mangancarbonat oder deren Gemische einsetzt.

5. Verfahren nach Anspruch 1, **dadurch gekennzeichnet, dass** man als Aktivierungsmittel ein Metallbromid oder ein organisches Bromid einsetzt.

6. Verfahren nach Anspruch 5, **dadurch gekennzeichnet, dass** das Metallbromid Natriumbromid oder Ammoniumbromid ist.

7. Verfahren nach Anspruch 5, **dadurch gekennzeichnet, dass** das organische Bromid Trietyl-*tert*-butylammonium-bromid ist.

8. Verfahren nach einem der Ansprüche 1 bis 7 **dadurch gekennzeichnet, dass** die Carbonsäure Eisessig ist.
